# EUROPEAN PATENT SPECIFICATION

(11) **EP 0 833 666 B1**
(45) Date of publication and mention of the grant of the patent: **10.12.2003**
(21) Application number: 97917902.5
(22) Date of filing: 09.04.1997
(51) Int. Cl.: A61K 47/48

(54) **CYTOMODULATING CONJUGATES OF MEMBERS OF SPECIFIC BINDING PAIRS**
ZELLMODULIERENDE KONJUGATE AUS ELEMENTEN AUS SPEZIFISCHEN BINDUNGSPAAREN
CONJUGUES CYTOMODULANTS D'ELEMENTS DE PAIRES DE LIAISON SPECIFIQUES

(30) Priority: 10.04.1996 US 630383
(43) Date of publication of application: 08.04.1998
(73) Proprietor: SANGSTAT MEDICAL CORPORATION, Menlo Park, CA 94025 (US)
(72) Inventor: POULETTY, Philippe, Atherton, CA 94027 (US)
(74) Representative: Baldock, Sharon Claire
(86) International application number: US9705842
(87) International publication number: WO97037690

(56) References cited:
- EP-A- 0 305 967
- EP-A- 0 324 625
- EP-A- 0 367 166
- EP-A- 0 369 387
- EP-A- 0 510 949
- WO-A-91/01146
- WO-A-91/07418
- WO-A-92/03569
- LUSSOW, A. R. ET AL: "Redirecting circulating antibodies via ligand-hapten conjugates eliminates target cells in vivo" J. IMMUNOTHER. EMPHASIS TUMOR IMMUNOL. (1996), 19(4), 257-265 CODEN: JIEIEZ;ISSN: 1067-5582, 1996, XP002050531
- SAHA, BHASKAR ET AL: "Toxic shock syndrome toxin-1-induced death is prevented by CTLA4Ig" J. IMMUNOL. (1996), 157(9), 3869-3875 CODEN: JOIMA3;ISSN: 0022-1767, 1996, XP002050532
- KUGE, SOICHI ET AL: "Superantigen-induced human CD4+ helper/killer T cell phenomenon. Selective induction of Th1 helper/killer T cells and application of tumor immunotherapy" J. IMMUNOL. (1995), 154(4), 1777-85 CODEN: JOIMA3;ISSN: 0022-1767, 1995, XP002050533
- LANDO, PETER A. ET AL: "T cell killing of human colon carcinomas by monoclonal-antibody- targeted superantigens" CANCER IMMUNOL. IMMUNOTHER. (1993), 36(4), 223-8 CODEN: CIIMDN;ISSN: 0340-7004, 1993, XP002050534
- LITTON, MARK J. ET AL: "Antibody-targeted superantigen therapy induces tumor-infiltrating lymphocytes, excessive cytokine production, and apoptosis in human colon carcinoma" EUR. J. IMMUNOL. (1996), 26(1), 1-9 CODEN: EJIMAF;ISSN: 0014-2980, 1996, XP002050535
- LUSSOW A R ET AL: "Targeting of activated T-cells with natural cytotoxic antibodies via an IL2-hapten conjugate prolongs graft survival." TRANSPLANTATION PROCEEDINGS, vol. 28, no. 2, April 1996, pages 571-572, XP002050536
- LUSSOW A R ET AL: "Targeting of antihapten antibodies to activated T cells via an IL-2-hapten conjugate prolongs cardiac graft survival." TRANSPLANTATION, vol. 62, no. 12, 27 December 1996, pages 1703-1708, XP002050537

## Description

### INTRODUCTION

### Technical Field

The field of this invention is therapeutics employing cytomodulating compounds.

### Background

The immune system is our major defense against a wide variety of diseases. However, in many situations, the immune system appears to be unable to protect the host from disease or is aberrant and attacks the host, being unable to distinguish between self and non-self. In both situations, there is an interest in being able to modulate the immune system, either activating the immune system toward a particular target or inactivating the immune system to prevent attack of a target.

For the most part, success in preventing immune system attack has relied upon the total inhibition or suppression of the immune system. In this situation, the host becomes susceptible to a wide variety of opportunistic infections. Therefore, while achieving one goal, one must protect the host against pathogens, which can result in extended periods of hospitalization, maintenance by antibiotics with the resulting side effects, and the like. By contrast, it is believed that the immune system is normally capable of protecting the host from tumorigenesis. However, the substantial incidence of cancer is evidence of the inability of the immune system to maintain perfect surveillance. In this situation, there is an interest in being able to activate the immune system so as to increase its capability to attack cancer cells.

There is, therefore, an interest in providing for therapies that can be specific for activating particular cells to be directed toward a specific target or inactivating specific cells which are directed to a specific target. In this way, one may selectively activate or inactivate members of the immune system to achieve a therapeutic goal.

### Relevant Literature

Lorberbaum *et al*. (1990) J. Biol. Chem. **265**:16311-7 describe a polyethylene-glycol modified IL-2. Batra *et al*. (1990) *ibid* **265**:15198-202 describe a fusion protein comprising a single chain antibody. Garrido *et al*. (1990) Cancer Res. **50**:4227-32 describe bispecific antibodies to target human T-lymphocytes. Pullen *et al*. (1990) Cell **61**:1365-74, describe the region of the T-cell receptor beta chain that interacts with the self-superantigen Mls.1A. Garrido *et al*. (1990) J. Immunol **144**:2891-8 describe targeted cytotoxic cells. Junghans *et al*. (1990) Cancer Res. **50**:1495-502 describe a humanized antibody to the IL-2 receptor. Schroeder *et a*/. (1990) Transplantation **49**:48-51 describe antimurine antibody formation following OKT3 therapy. Kaplan and Mazed (1989) Int. J. Artif. Organs **12**:79-804 describe *in vitro* removal of anti-A and anti-B antibodies with synthetic oligosaccharides. A review of blood group antigens may be found in FEMS Microbiol. Immupol. (1989) **1**:321-30.

Ochi *et al*. (1993) J. Immunol. **151**:3180-3186 describe the use of a conjugate of SEB-anti-tumor antibody conjugates for tumor immunotherapy.

### SUMMARY OF THE INVENTION

Conjugates of selective binding moieties are used to modulate immune response. The conjugates, referred to as "complexines" comprise a first moiety that binds to a target, usually a cell receptor bound to the membrane of a cell or a soluble molecule, particularly in circulation, and a second moiety that binds to an effector agent endogenous to the host, which provides for cytomodulation, normally cytotoxicity. The complexine is administered to the host in amounts sufficient to provide for the desired prophylactic or therapeutic effect.

### BRIEF DESCRIPTION OF THE DRAWINGS

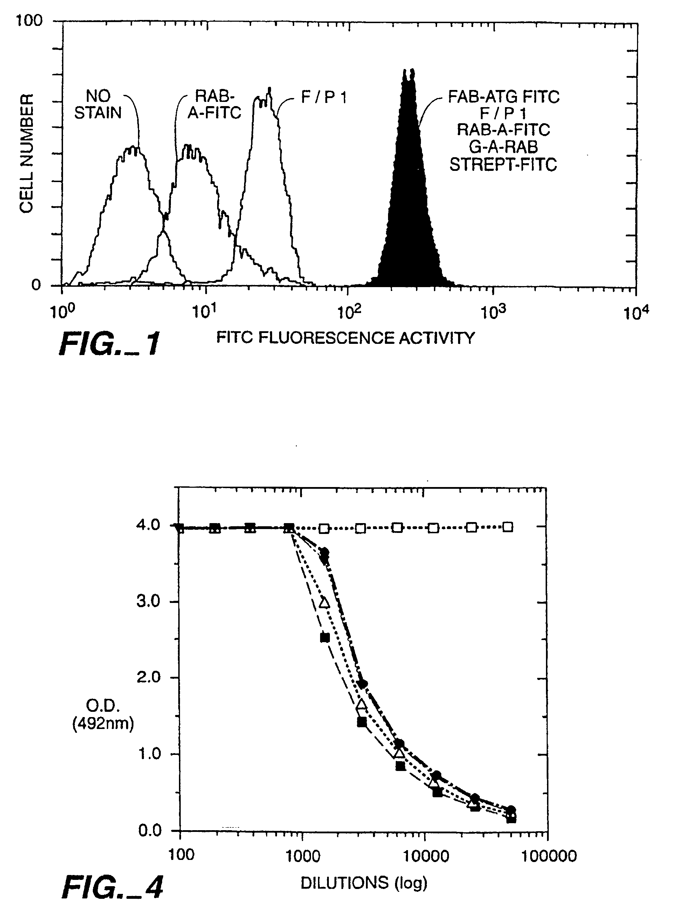

Figure 1 shows evidence that complexine and anti-FITC antibodies can be demonstrated on the cell surface of P815 cells analyzed by flow cytometry with either no antibodies on the surface (no stain peak), the addition of F(ab¹)₂ATG-FITC (F/P 1 peak), or after the indirect labeling of the rabbit anti-FITC antibodies with a biotinylated goat anti-rabbit immunoglobulin and streptavidin bound FITC (solid peak).

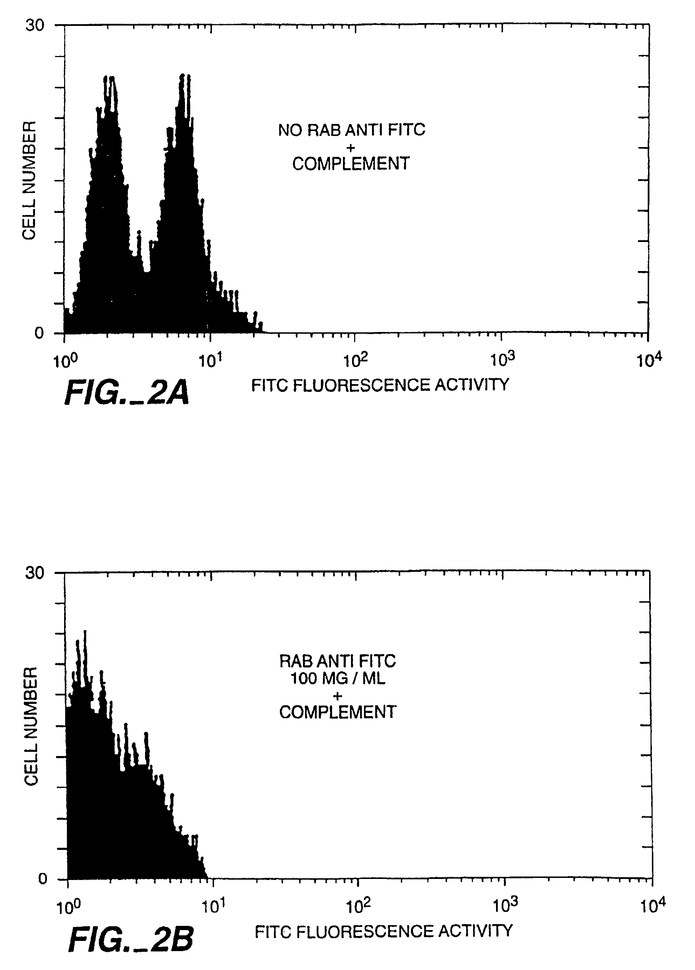

Figure 2A and Figure 2B show data that anti-FITC antibodies bound to the cell surface via complexine induce lysis in the presence of complement. Figure 2A illustrates that there is no effect of complement on the two populations in the absence of anti-FITC antibodies. Figure 2B illustrates that once the complexine on the cell surface has bound to the anti-FITC antibodies in the presence of complement there is lysis of the labeled population, as evidenced by the disappearance of the stained population.

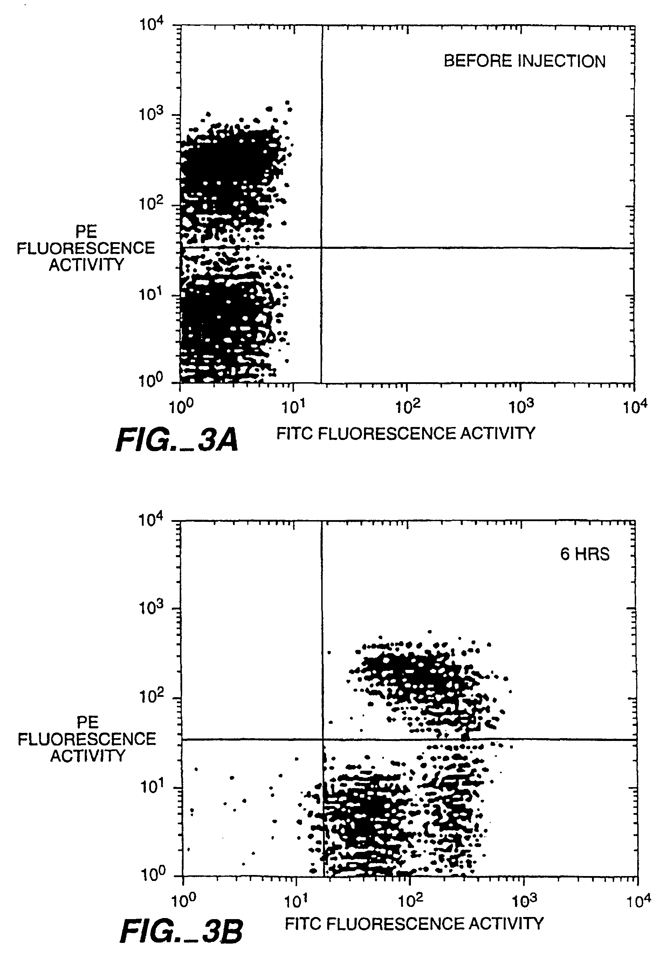

Figure 3A and Figure 3B show that complexine can be demonstrated on cells *in vivo*. Figure 3A shows PBLs separated from the blood of mice before injection of 250 µg of F(ab¹)₂ATG-FITC and Figure 3B shows PBLs separated from the blood of mice 6 hrs after injection of 250 µ9 of F(ab¹)₂ATG-FITC. The cells were stained with anti-CD3-PE to identify the T-cell population.

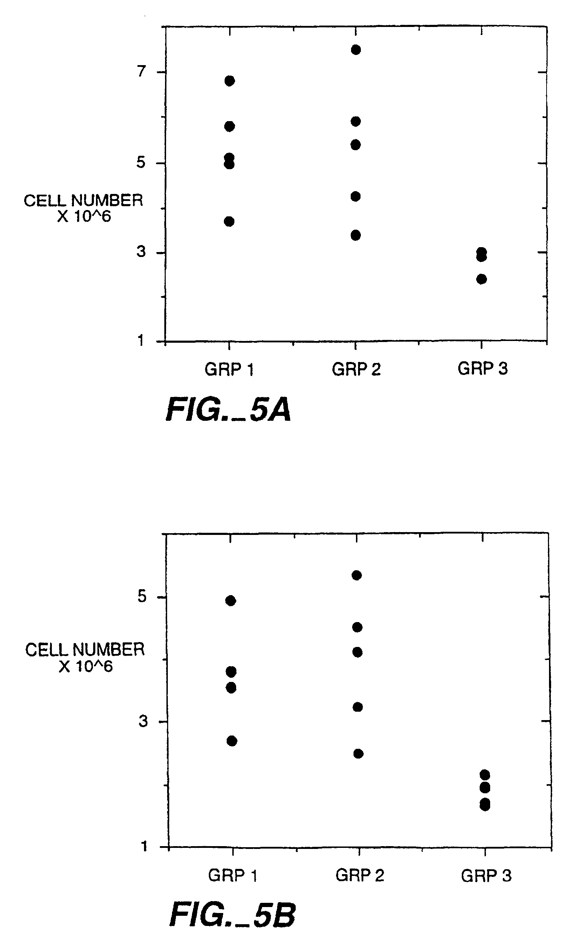

Figure 4 demonstrates that anti-FITC antibodies can be detected circulating in the mouse long after their injection. Five mice (solid lines, a different symbol for each mouse) were injected with 1 mg of rabbit anti-FITC antibodies, and 48 hrs later blood was drawn and assayed for the presence of rabbit antibodies by ELISA. Serum from a BSA-FITC immunized rabbit (dotted lines, 12/28/93) was used as a positive control.
Figure 5A and Figure 5B are graphs demonstrating that administration of complexine to mice transfused with anti-FITC antibodies leads to the decrease of circulating PBLs and T-cells. Three groups of five mice were treated as follows: Grp 1 received 1 mg of anti-FITC antibodies at time 0 and 200 µl of PBS 24 hrs later. Grp 2 received PBS and then 250 µg of F(ab¹)₂ATG-FITC. Grp 3 mice were transfused with 1 mg of anti-FITC antibodies at time 0, and 250 µg of F(ab¹)₂ATG-FITC 24 hrs later. Figure 5A shows the number of circulating PBLs and Figure 5B shows the number of T-cells 24 hrs after the last injection.

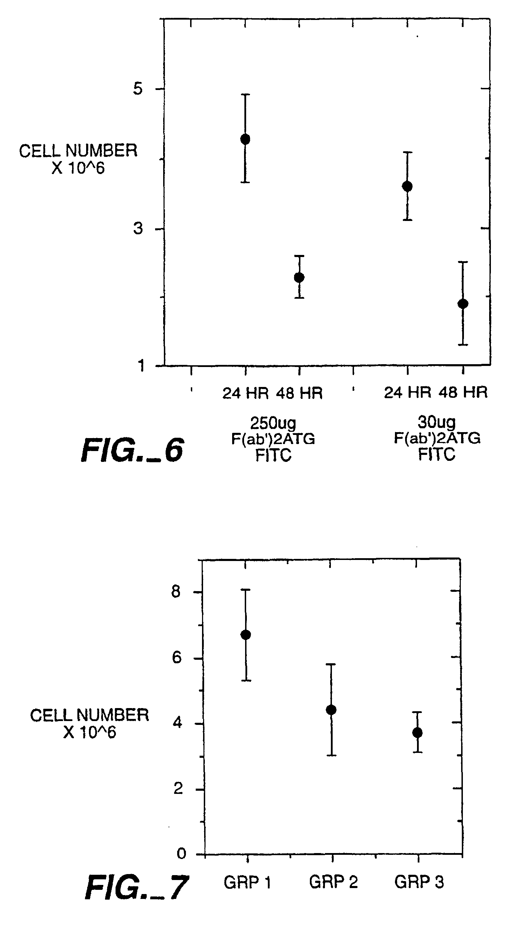

Figure 6: Low doses of complexine are effective at depicting the CD3+ cell population. Two groups of five mice received 250 µg of purified anti-FITC antibodies i.v.. The numbers of circulating CD3+ cells were evaluated 24 hours later. These data were compared with the numbers of CD3+ cells after the injection of either 250 or 30 µg of F(ab¹)₂ATG-FITC (t=48 furs: mean and standard deviation shown here). The low dose of complexine proved as effective as the high dose in eliminating the target cell population.
Figure 7: Complexine eliminates cells *in vivo* as well as conventional therapies. In order to compare the efficacy of complexine treatment in the subject model with conventional methods of cell depletion, equivalent doses of complexine and ATG were administered. Grp1 mice received 250 µg of anti-FITC antibodies at t=1 and 50 µg of F(ab¹)₂ATG at t=24 hrs. Grp2 mice were injected with the same dose of anti-FITC antibodies at t=0, and with 50 µg of F(ab¹)₂ATG-FITC at t=24 hrs. The mice in Grp3 received 50 µg of ATG only at t=24 hrs. The numbers of circulating PBL (mean and standard deviation) at t=48 hrs are represented here. There was no measurable difference between the decrease in cell number following complexine administration (Grp2) and ATG administration (Grp3:p<=0.05, Scheffe ANOVA).

### DESCRIPTION OF THE SPECIFIC EMBODIMENTS

Compositions are provided for therapeutic treatment of a host, where the action of the immune system is modulated, so as to provide for prophylactic or therapeutic effect. The method employs a conjugate having two moieties, each moiety having physiological activity. One moiety provides for binding to a target on a target cell or a soluble molecule, particularly in circulation. The other moiety provides for interaction with a member of the immune system, whereby endogenous agents provide for the prophylactic or therapeutic effect. The conjugates may be as a result of chemical binding, either covalent or non-covalent, or a fusion protein by means of genetic engineering. Thus, the complexine components may be held together by means of a synthetic bridge, a peptide bridge, a membrane, *e.g.* liposome, polymer or particle, etc.

The conjugates are called "complexines", since they result in the formation of complexes with members of the immune system, which provide for modulation of the activity of a target cell. By administering the complexines to a host, the complexines will bind to a target epitope, usually a surface membrane protein of a target cell or a soluble molecule that has adverse physiological effects, while also binding to an endogenous effector agent present in the host. The endogenous effector agent results in an endogenous pathway for inactivation or removal of the target from the host. For the most part, with cells cytotoxicity is obtained, whereby a target cell is killed. For molecules, complexes are formed that are eliminated.

The moiety that binds to the target cell receptor or soluble molecule may be any of a wide variety of molecules, including immunoglobulins, fragments thereof, including heavy chains, light chains, Fab, F(ab¹)₂, Fc, either monoclonal or polyclonal, and the like; anti-idiotype antibodies, which simulate a ligand; ligands for surface membrane receptors or fragments thereof, such as the interleukins 1-16, particularly - 2, -4 and -6, or folate, which binds to high affinity receptors expressed at an elevated level on many tumor cells; molecules that bind to cluster designation surface membrane proteins, such as CD3, -4 , -5, -8, -10, -15, -19, -69, etc.; growth factors, such as granulocyte-macrophage colony stimulating factor (GM-CSF), granulocyte colony stimulating factor (G-CSF), macrophage colony stimulating factor (M-CSF), epidermal growth factor (EGF), tumor growth factor (TGF), tumor necrosis factor (TNF), interferons, etc.; molecules that bind to any of the members of the T-cell receptor, either the sub-units of Tᵢ or T₃; slg; molecules that bind to infectious agents *etc*.; molecule that bind to LPS, or other pathogenic cellular marker; molecules that bind to bacterial receptors, *etc*.; in the case of transplantation of organs, HLA molecules or fragments derived thereof from donor HLA antigens, particularly the variable region, while for bone marrow transplants the HLA molecules will be from the recipient antigens, *etc*.

Small organic molecules that specifically bind to the target cell receptor or soluble molecule are of interest as a binding moiety. Such molecules generally have a molecular weight of more than 100 and less than about 5000 daltons and comprise functional groups that structurally interaction with proteins, particularly hydrogen bonding, and typically include at least an amine, carbonyl, hydroxyl or carboxyl group, preferably at least two of the functional chemical groups. Such molecules often comprise cyclical carbon or heterocyclic structures and/or aromatic or polyaromatic structures substituted with one or more of the above functional groups. They are obtained from a wide variety of sources including libraries of synthetic or natural compounds. Libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. See, for example, Seelig *et al*. (1994) J. Biol. Chem. **269**:358-363; and Vaughan *et al*. (1996) Xenotransplantation **3**:18-23.

For soluble molecule targets, one may use various binding moieties with high affinity for the soluble molecules, such as antibodies, mono- or polyclonal, fragments thereof, e.g., Fab, Fv, F(ab¹)₂, *etc*., modified antibodies, *e*.*g*. humanized mouse antibodies, or the like, lectins, enzymes, surface membrane protein receptors, or other specific binding entity. Targets may include interleukins and cytotokines, *e*.*g*. IL-2 and -6, TNF-α, *etc*., bacterial toxins, autoimmune antibodies, hormones, *e*.*g*. estrogens, *etc*.

The other moiety of the conjugate is a selective member, where the member directly or indirectly binds selectively to an effector system, comprising one or more effector agents endogenous to the host. By endogenous is intended an agent which is naturally present or may be safely administered to or induced in the host, *e*.*g*. antibodies, so as to be able to react with the selective members. Of interest are antibodies that are commonly found at high levels, such as antibodies specific for the α-galactosyl epitope. The selective member may be an antigen to which the host has been previously sensitized or to which the host has natural antibodies, so as to have memory cells and/or specific soluble antibodies in the blood stream, such as oligosaccharide A or B antigens, vaccine antigens (immunogens) which encounter antibodies due to a prior immune response, *e*.*g*. diphtheria or tetanus antitoxin, influenza virus hemagglutinin, HBs antigen, polio virus, rubella virus or measles virus antibodies, such as antibodies to DNA, RNA or ribonucleoprotein. The selective member for a T-cell response may be tuberculin, HIV, particularly gp120, a superantigen, such as toxins derived from Staphylococcus or other bacteria, *e*.*g*., SEC1, SEA, SEB, ExFT, TSST1, Mls, or minor histocompatibility antigens from mammalian cells. The superantigens bind to a substantial proportion of the Vβ chains of the T-cell receptor, Ti. In all cases, one may use various groups that provide the same function, *e*.*g*. binding. Of interest are anti-idiotype antibodies or the variable regions thereof, which will mimic the selective moiety or bind to antibodies present in the host. The antibodies may interact with the members of the complement cascade or other cytotoxic agent, *e*.*g*. ADCC, to kill the target cell or the selective member may bind to a T-cell that provides a cytotoxic function.

The selective member is anti-α-galactosyl antibodies. This antibody is reported at levels of 1% of the total IgG percent in human blood. See Galili *et al* (1985) J. Exp. Med. **162**:573-582; Galili *et al*. (1987) Proc. Natl. Acad. Sci. USA **84**:1369-1373; and Galili *et al*. (1993) Blood **82**:2485-2493. The ligand for the antibody is the epitope Gal α1-3 Gal β-1-4 GIcNAc-R, referred to as the α-galactosyl epitope. In reference to the "galactosyl epitope" is intended any compound that specifically binds to an antibody specific for α-galactosyl, including combinatorially derived mimetics (see Vaughan *et al., supra*.) The epitope has been conjugated to beads (Chembiomed, Edmonton Alberta), can be readily synthesized and may be conjugated to the moiety binding to the target cell in conventional ways. Because subclasses of IgG participate in the complement cascade and ADCC, linking the α-gal epitope to the target cell binding moiety results in cytotoxicity of the target cell upon binding of the complexine conjugate to the target cell. For example, the α-galactosyl epitope may be conjugated to folate, which binds to tumor cells with high efficiency. Upon injection of a complexine bearing the α-gal epitope, the complexine will bind to the target and also to the α-gal antibody. If the target member is a cell, the α-gal antibody will initiate the complement cascade or mediate ADCC, resulting in lysis of the target cell. If the target is a soluble molecule, the immune complex involving the α-gal antibody will result in clearance of the target molecule.

The members of the conjugate may be polypeptides, saccharides, lipids, nucleic acids, or naturally occurring or synthetic organic molecules other than the molecules already described. The members of the conjugate may be joined directly or through a bridge of not more than about 50 members in the chain, usually not more than about 20 members in the chain, where the members of the chain may be carbon, nitrogen, oxygen, sulfur, phosphorous, and the like. Thus, various techniques may be used to join the two members of the conjugate, depending upon the nature of the members of the conjugate, the binding sites of the members of the conjugate, convenience, and the like. Functional groups that may be involved include esters, amides, ethers, phosphates, amino, hydroxy, thio, aldehyde, keto, and the like. The bridge may involve aliphatic, alicyclic, aromatic, or heterocyclic groups. A substantial literature exists for combining organic groups to provide for stable conjugates. Conjugates involving only proteins or glycoproteins can be chimeric or fusion recombinant molecules resulting from expression of ligated open reading frames of natural sequences, synthetic sequences, or combinations thereof.

In the embodiment of the invention utilizing naturally occurring anti-α-gal antibodies, the α-galactosyl epitope is conjugated to the moiety that binds to the target cell receptor or soluble molecule. Depending upon the nature of the chemistry, the α-galactosyl group may be introduced in association with one or more groups Various chemistries may be employed for joining the galactosyl epitope to a variety of functionalities. See, for example, Gobbo *et al*. (1992) Int. J. Pept. Protein Res. **40**:54-61; Wood and Wetzel (1992) Bioconjug. Chem: **3**:391-6; Filira *et al*. (1990) Int. J. Pept. Protein Res. **36**:86-96; Kazimierczuk *et al*. (1985) Z. Naturforsch. **40**:715-720; Rademann and Schmidt (1995) Carbohydr. Res. **269**:217-25; and Wong *et al*. (1993) Glycoconj. J. **10**:227-234. The particular manner in which the α-galactosyl epitope is joined to the binding moiety is not critical to this invention, so long as the α-galactosyl epitope is available for binding to antibodies in the blood.

The ratio of conjugate member to the moiety that binds to the target cell receptor or soluble molecule may vary. In some situations, it may be desirable to have more than one conjugate member per ligand moiety to provide for higher avidity or activity or vary the *in vitro* solubility of the complex or for extended immune complexes and/or more than one ligand moiety per conjugate, for similar reasons. Generally, the ratio of conjugate member to ligand moiety will be less than about 20, usually less than about 3, frequently 1. Higher ratios may be used where the conjugate members do not interfere with with the physiological activity of the complexine.

Illustrative of complexines are IL-2 or CD69 binding protein for binding to T-cells individually linked to polysaccharide A and polysaccharide B antigen as a mixture, where the complexine comprises individual A and B molecules. Since about 95% of individuals have natural anti-A and/or anti-B antibodies, these complexines will be effective in about 95% of individuals. Thus, one could have a combination of IL-2, IL-4 and/or CD69 binding protein or combination of selective moieties *e*.*g*. A + B + vaccine Ag.

The multivalent complexine is devised so that when administered intravenously it is soluble in the circulation and will bind to T-cells expressing the target surface membrane protein. If one wishes to destroy activated T-cells which have a high level of IL-2 receptor or express CD69, the subject complexines will serve to bind via antibodies to the selective moiety to complement or other cytotoxic agent, such as ADCC cells, to substantially reduce the activated T-cell population. The binding of the effector antibodies to complexines on the target cells will result in complement activation and/or opsonization resulting in target cell lysis. Other effector agents, include lymphocytes, or neutrophils, such as T-cells or K-cells, NK cells, monocytes, macrophages, basophils, eosinophils, mastocytes, erythrocytes, *etc*. By employing superantigen selective for cytotoxic T-cells, one may recruit such cells for their cytotoxic effect.

The subject compositions may be used for the treatment of a wide variety of pathologies by varying the moiety for the target cell. Thus, treatments may include immunosuppression for organ transplantation, treatment for neoplasias such as carcinomas, leukemias, lymphomas, sarcomas, melanomas, *etc*., autoimmune diseases such as rheumatoid arthritis, multiple sclerosis, systemic lupus erythematosus, *etc*.; cellular pathogens, such as bacteria; and the like. Unique specificity is not required as long as there is a substantial preference for binding to the target cells.

One example is immune suppression associated with organ transplantation. In this situation, one would wish to inactivate or destroy T-cells that are active against the organ transplant. Thus, those T-cells that are activated and have high levels of IL-2 receptor or recognize the HLA antigen may be selectively targeted for destruction by use of a complexine comprising one or more IL-2 ligands or binding portion thereof or other ligands, *e.g.* other interleukins, or one or more HLA antigens or the variable regions thereof of the organ donor. The IL-2 ligand may be conjugated to a moiety such as FITC, α-gal, *etc*. In the case of a bacterial infection, one may use a lectin or antibody specific for an epitopic site of the pathogen, bonded to the A and/or B antigen, or α-gal, to enhance the immune response to the pathogen.

The subject conjugates will for the most part be administered parenterally, particularly intravascularly, topically, as an aerosol, orally or the like, depending upon the particular organ, system or chamber to be treated. The amount of the conjugate that is administered will vary widely, depending upon the nature of the conjugate, the nature of the disease being treated, whether one or more administrations are to be made, the endogenous level of the effector or level stimulated by the complexine, the desired cytotoxic level, and the like. Thus, for each conjugate, one will determine empirically the level to be administered for a particular indication. The conjugates may be administered in any convenient carrier, such as distilled water, phosphate buffered saline, saline, aqueous ethanol, blood derivative, or other conventional carrier. Other additives may be included, such as stabilizers, biocides, buffers, salt, and the like, these additives being conventional and used in conventional amounts. For example, with mice, injections of complexines comprising F(ab¹)₂-ATG employ amounts in the range of about 10-500 µg.

The following examples are by way of above illustration and not by way of limitation.

### EXPERIMENTAL

### Example 1

### Complexine with red blood Group A antigen.

Blood-group A synthetic trisaccharides (8-azidocarbonyloctyl-derivatives of alphaGalNacl, 3-[alphaFuc 1,2]betaGal derivatized to include a C-terminal amino group is conjugated to Interleukin 2 as follows:
*Activation of Interleukin 2*: 0.2 mg (13 nmoles) of IL-2 is dissolved in 0.3 ml of 0.1 M sodium phosphate pH 7.5. 2.3 mg N-Succinimidyl S-Acetyl thiolacetate is dissolved in 1 ml of DMSO. 10 µl of N Succinimidyl S-Acetyl thiolacetate is added to the IL-2 solution and the mixture is incubated at 25°C for 30 minutes. The reaction mixture is desalted by passage through a SEPHADEX G-25 column equilibrated with 0.1 M phosphate buffer pH 6.0. Free thiol groups are formed by adding 100 µl of 0.5 M hydroxylamine/0.05 M sodium phosphate pH 7.5 containing 0.025M EDTA to the IL2 solution. The solution is stirred for 120 minutes at room temperature. The solution is again passed through a G-25 column and IL-2 bearing free SH groups is obtained. Free SH groups are quantified using the Ellman's test, by measuring the absorbance at 412 nm.
*Activation of Blood Group A Trisaccharide (A antigen) With Maleimido Group:* A antigen (0.50 to 2 x molar excess of IL-2) is dissolved in 1.0 ml of 0.1 M sodium phosphate pH 7.5. Succinimidyl N-Maleimido-6-aminocaproyl (2nitro-4-sulfonic acid) phenyl ester Na (MALSAC-HNSA) (100 molar excess) is dissolved in 20 µl of dimethyl sulfoxide (DMSO). The reagent solution is added to the A antigen solution and the mixture is incubated at 25°C for 1 hour. The reaction is monitored by diluting 10 µl of the reaction mixture into 1.0 mL of 0.01 M sodium phosphate, pH 7 at timed intervals. Each aliquot is analyzed by reading the absorbance at 406 nm (A406) before and after addition of 50 µl of 5 N NaOH. The percentage of active ester at any time is calculated using the formula:

   [(A406(NaOH) -A406)/A406(NaOH)] x 100.

   From the difference between the amount of ester at t₀ and at a time thereafter, the amount of ester used at that time is calculated. That corresponds to the amount of total amino group modified. The reaction mixture is centrifuged briefly to remove excess of precipitated reagent and the supernatant is applied to a Sephadex G-25 column equilibrated in 0.1 M phosphate pH 6.0.
*Conjugation (A-Complexine)*: To maleimido-A antigen (in 0.1 M phosphate pH 6.0), is added IL-2-SH compound and allowed to stir at 4°C overnight. The final molar ratio of Mal-A antigen and IL-2-SH is adjusted from 0.2 to 5. The reaction mixture is then passed through Sephacryl-200. The reactions having peaks at desired mol. weight ranges are collected. Antigenic reactivity of the conjugate is tested by Western blot using anti-blood group antigen human serum and anti-IL2 monoclonal antibody.
*Functional Assay of A-Complexine*: In this experiment, it is determined whether the A-Complexine can be used *in vitro* to induce the specific killing of lymphocytes expressing a high affinity IL-2 receptor, when mixed with human serum containing anti-blood group A antibodies and complement. ⁵¹Cr labelled CTLL-2 lymphocytes are incubated with A-Complexine (from 40 µg/mL to 0.1 µg/mL). After 45 min. incubation at 37°C, human serum containing anti-blood group A antibodies (B group) is added at various dilutions and incubated for 30 minutes at 37°C; rabbit complement is then added and incubated 1 hour at 37°C. The amount of ⁵¹Cr released is then estimated and the percentage of specific cell lysis calculated. Significant lysis of CTLL-2 cells is observed after incubation with A-Complexine. The phenomenon is dose dependent (increased cytotoxicity), with higher amounts both of A-Complexine and anti-blood group A positive serum) and specific, as IL2-receptor negative cell lines (such as DA-la mouse cells) are not killed under the same assay conditions. Furthermore, no significant cytotoxicity is observed when using human serum from a blood group AB or A individual.

### Example 2

### HBs Complexine

A cyclical peptide derived from the amino-acid sequence (a.a. 139-147) of Hepatitis B virus surface antigen (HBsAg) and showing antigenic reactivity with polyclonal and monoclonal antibodies defining the a epitope of HBsAg (HBs peptide) is used for conjugation with interleukin 2. The peptide sequence is: NH2-Cys-Thr-Lys-Pro-Thr-Asp-Gly-Asn-Cys-Tyr-COOH. It is synthesized by solid phase method (Merrifield) using FMOC chemistry. It is purified by HPLC. A disulfide bond is introduced between the two terminal cysteine residues by oxidation with potassium ferricyanide.

*Synthesis*: HBs peptide is conjugated to Interleukin 2 as follows:
*Activation of Interleukin 2*: 0.2 mg of (13 nmoles) of IL-2 is dissolved in 0.3 ml of 0.1 M sodium phosphate pH 7.5. 2.3 mg N-Succinimidyl S-Acetyl thiolacetate is dissolved in 1 ml of DMSO. 10 µl of N-Succinimidyl S-Acetyl thiolacetate is added to the IL-2 solution and the mixture is incubated at 25°C for 30 minutes. The reaction mixture is passed through a G-25 column equilibrated with 0.1 M phosphate buffer pH 6.0. Free thiol groups are introduced by adding 100 µl of 0.5 M hydroxylamine/0.05 M sodium phosphate pH 7.5 containing 0.025 M EDTA to the IL-2 solution. The solution is stirred for 120 minutes at room temperature. The solution is passed through a G-25 column and free SH groups on IL-2 are obtained. Free SH groups are quantified using the Ellman's test, by measuring the absorbance at 412 nm.
*Activation of HBs Peptide With Maleimido Group*: HBs peptide (0.50 to 2 x molar excess of IL-2) is dissolved in DMSO at 10 mg/ml and diluted in 1.0 ml of 0.1 M sodium phosphate pH 7.5. Succinimidyl N-Maleimido-6-aminocaproyl (2-nitro-4-sulfonic acid) phenyl ester Na (MAL-SAC-HNSA) (100 molar excess) is dissolved in 20 µl of dimethyl sulfoxide (DMSO). The reagent solution is added to the HBs peptide solution and the mixture incubated at 25°C for 1 hour.

The reaction is monitored by diluting 10 µl of the reaction mixture into 1.0 ml of 0.01 M sodium phosphate pH 7 at timed intervals. Each aliquot is analyzed by reading the absorbance at 406 nm (A₄₀₆) before and after addition of 50 µl of 5 N NaOH. The percentage of active ester at any time is calculated using the formula:

[(A₄₀₆(NaOH)-A₄₀₆)/A₄₀₆(Na/OH)] x 100

From the difference between the amount of ester present at t₀ and at a time thereafter, the amount of ester used at that time is calculated. That corresponds to the amount of total amino group modified. The reaction mixture is centrifuged briefly to remove the excess of precipitated reagent and the supernatant is applied to a Sephadex (G-25 column equilibrated in 0.1 M phosphate pH 6.0).

*Conjugation*: To maleimido-HBs peptide (in 0.1 M Phosphate pH 6.0) is added IL-2-SH compound and allowed to stir at 4°C overnight. The final molar ratio of Mal-HBs peptide and IL2-SH are adjusted from 0.2 to 5. Finally the reaction mixture is passed through Sephacryl-200. The peaks at the desired molecular weight ranges are collected. Antigenic reactivity of the conjugate is tested by western blot using anti-HBs monoclonal antibody (A specific) and anti-IL-2 monocolonal antibody.

*Functional Assay of Hbs-Complexine*: In this experiment it is determined whether the HBs-Complexine can be used to induce *in vitro* the specific killing of lymphocytes expressing a high affinity IL-2 receptor, when mixed with human serum containing anti-HBs antibodies and complement. ⁵¹Cr labelled CTLL-2 lymphocytes are incubated with HBs-Complexine (from 20 µg/mL to 0.1 ng/mL). After 45 min. incubation at 37°C, human serum containing anti-HBs antibodies (collected from a patient vaccinated using Hevac B, Pasteur Vaccins and tested for anti-HBs antibodies by ELISA (Abbott Laboratories)) is added at various dilutions, incubated for 30 minutes at 37°C; rabbit complement is then added and incubated 1 hour at 37°C. The amount of ⁵¹Cr released is then estimated and the percentage of specific cell lysis calculated. Significant lysis of CTLL-2 cells is observed after incubation with Hbs-Complexine. The phenomenon is dose dependent: increased cytotoxicity is observed both with higher amounts of HBs-Complexine and anti-HBs positive serum. Cytotoxicity is specific, as IL-2 receptor negative cell lines (such as DA-la mouse cells) are not killed under the same assay conditions. Furthermore, no significant cytotoxicity is observed when using human serum negative for anti-HBs antibodies.

### Example 3

### Preparation of Folate α-Gal Complexines

### Materials and Methods

*Anti-α-Gal antibodies: purification and murine cell recognition*: Human anti α-Gal antibodies are purified from pooled plasma using a melibiose agarose column (Sigma Chemical Co., St. Louis, MO). Briefly, plasma from normal blood-donors -is passed over the column, the support washed with PBS, and the bound antibodies eluted by adding 0.1M Tris pH 4.0. Protein concentration in the eluted fractions is measured (BCA test; Pierce, Rockford, IL), and the fractions containing protein are pooled.

*Preparation of Folate conjugates*: folate is coupled through carboxyl groups to anti α-antibody amine groups by a carbodiimide procedure. A five-fold molar excess of 1-ethyl-3-(3-dimethylaminopropyl)carbodiimide hydrochloride (EDC) is added to folate dissolved in dimethyl sulfoxide. After 30 minutes at room temperature in the dark, a 10- or 100-fold molar excess of the EDC activated folate is added to the 0.5-2.0 mg of antibody in 0.1M MOPS, pH 7.5. After 1 hour at room temperature, the sample is applied to a sephadex G-25 column equilibrated in phosphate-buffered saline (10 mM NaPO₄/150 mM NaCl). The excluded peak fractions are pooled and analyzed spectrophotometrically at 280 and 363 nm. Epitope density of the folate on antibody conjugates are determined by using molecular extinction coefficients for folate of 6,197 (363 nm) and 25,820 (280 nm). Antibody concentrations are determined by subtracting the absorbance contribution of folate at 280 nm and by using an antibody extinction coefficient of 224,000.

*Folate Binding Assays*: Binding assays are conducted using ¹²⁵1-labeled folate. Cells are washed with PBS containing 0.1% BSA to remove excess free folate. Cells, labeled folate and competitors are incubated in PBS-BSA for 1 hour. Bound and free ligand are separated by centrifugation.

*Functional Assay of Folate-α-Gal Complexine*: It is determined whether the folate-α-gal complexine can be used to induce *in vitro* the specific killing of tumor cells expressing a high affinity folate receptor when mixed with normal human serum. ⁵¹Cr labelled tumor cells known to express high levels, of folate receptor are incubated with folate-α-gal complexine (from 20 µg/mL to 0.1 ng/mL). After 45 min. incubation at 37°C, normal human serum is added at various dilutions, incubated for 30 minutes at 37°C; rabbit complement is then added and incubated 1 hour at 37°C. The amount of ⁵¹Cr released is then estimated and the percentage of specific cell lysis calculated. Lysis of tumor cells is observed after incubation with folate-α-gal complexine.

### Example 4. Activity of a Complexine in vivo

A mouse system was designed and tested to illustrate the therapeutic potential of complexines. In these experiments, a F(ab¹)₂ fragment of polyclonal anti-thymocyte globulin (ATG) preparation is bound to fluorescein isothiocyanate (FITC) and used as the complexine construct. Coupling of the fluorescent molecule to the F(ab')2 fragment as described below does not prevent binding of the antibody to cells. An anti-FITC antibody preparation will recognize the complexine on the surface of cells. Further, the combination of anti-FITC antibodies and complexine at the surface leads to cell lysis in the presence of complement *in vitro*. In order to rapidly establish an *in vivo* environment containing high titer anti-fluorescein antibodies, mice are passively transfused with anti-FITC antibodies. The administration of complexine to these mice results in a more than 50% decrease in the numbers of their circulating T-cells. This demonstrates that cytomodulating conjugates of members of specific binding pairs in a mammalian system have activity *in vivo*.

### Materials and Methods

*Complexine*: F(ab¹)₂ fragments of ATG (Accurate Chemical and Scientific Corp., Westbury, N.Y.) are prepared by standard protocols. ATG antibodies are diluted to 1 mg/ml in 20 mM sodium citrate pH 3.5 and cleaved during a 90 min. incubation at 37°C with 5 µg/ml pepsin (Sigma Chemical Comp., St. Louis MO). Carbonate buffer pH 9.5 is added to a final concentration of 0.05 M in order to stop the reaction. The solution is then centrifuged over a filter in a centriprep-10 concentrator (Amicon, Beverley, MA) to a final protein concentration of 20 mg/ml.

The F(ab¹)₂ ATG fragment is then coupled to FITC in the following manner. 120 µg of FITC (fluorescein 5-isothiocyanate isomer 1 from Sigma Chemical Comp.: stock solution at 20 mg/ml in DMSO) is added to 3 ml of the F(ab¹)₂ solution, and incubated at room temperature for 30 min in the dark. Excess FITC is removed by passing the solution over a Sephadex G10 column (Pharmacia, Uppsala, Sweden). The protein content in the complexine is measured using a standard bichinoic acid based test (BCA test from Pierce, Rockford, IL), and a ratio of FITC/protein (F/P) equal to 1 in the conjugate is determined as described in Goding, J. W., Monoclonal Antibodies, 1986 Academic Press, San Diego, CA.

*Anti-FITC antibodies*: Bovine serum albumin (BSA: Boehringer Mannheim Corp., Indianapolis, IN) is conjugated to FITC. Two New Zealand White rabbits (purchased, maintained and manipulated at EL Labs, Soquel, CA) are immunized once with 100 µg of the BSA-FITC conjugate in Freund's complete adjuvant (Sigma Chemical Comp.) and twice thereafter with 100 µg of the BSA-FITC in incomplete Freund's adjuvant (Sigma Chemical Comp,). All immunizations are delivered subcutaneously at multiple sites, and the animals are bled by venous puncture once every two weeks.

Serum from the immunized rabbits is pooled and passed over a FITC bound column prepared according to the manufacturers instructions (Pharmalink kit, Pierce). Rabbit antibodies specific for the FITC are eluted from the column with immunopure IgG elusion buffer (Pierce). The protein content of the eluted fraction is then measured in the BCA assay (Pierce).

*Anti-FITC ELISA*: Keyhole limpet hemocyanin (KLH, Pierce) is coupled to FITC using the same procedure described for preparation of the F(ab¹)₂ ATG conjugate. 100 µg of KLH-FITC per plate is then coated on 96 well maxisorp (Nunc, Naperville IL) plastic plates overnight at 4°C. Unbound sites on the plastic are then saturated with a PBS-5% dehydrated non-fat milk (PBS-milk) solution for 1 hr at 37°C. After washing three times with PRA wash (SangStat Med. Corp., Menlo Park, CA) serial dilutions of samples in PBS-2.5% milk are added to each well and incubated for 1 hr at 37°C. The plates are again washed, then incubated with an anti-rabbit-HRP conjugate (Jackson ImmunoResearch, West Grove, PA). Following another incubation for 1 hr at 37°C and wash, the presence of rabbit anti-FITC antibodies is revealed by adding a 3 mg/ml solution of o-phenylenediamine dihydrochloride (Sigma Chemical Comp.) in substrate buffer (SangStat Med. Corp.). The enzymatic reaction is stopped after 15 min. by the addition of 100 µl 1N HCl and the results evaluated at 495 nm using an Emax spectrophotometer and SoftMax software (Molecular Devices, Menio Park, CA).

*FACS analysis*: Cultured P815 or peripheral blood lymphocytes (PBLs) are separated from heparinized whole blood on a ficoll gradient (Histopaque, Sigma Chemical Corp.) by centrifugation and washed in PBS containing 5% goat serum. Cells are then resuspended in 50 µl of the appropriate antibody and incubated for 20 min on ice. The T-cell receptor is detected using an anti-CD3-PE (Pharmingen, San Diego, CA). The presence of rabbit anti-FITC antibodies bound to complexine on the cell surface *in vitro* is revealed by the addition of biotinylated goat anti rabbit Fc 1g antibodies (Jackson ImmunoResearch, West Grove, PA) followed by the addition of streptavidin coupled FITC (Molecular Probes, Eugene, OR). All samples are analyzed using a FACScan and the Lysys II software (Becton Dickinson, San Jose, CA).

*Complement mediated lysis in vitro*: P815 cells are labeled with complexine as for FACS analysis then mixed with an equal number of unlabeled cells. Next, rabbit-anti FITC antibodies are added, as for FACS staining. After washing in serum free PBS the cells are incubated for 1 hr at 37°C in a 1:10 dilution of rabbit class I complement (One Lambda, Canoga Park, CA). The cells are washed, resuspended, then analyzed by FACS for a decrease in the numbers of FITC bearing cells.

*Mice and injections*: Groups of five BALB/c female mice 9-10 weeks of age (Simonsen Laboratories, Gilroy, CA) are used throughout the experiments. All injections are prepared in 200 µl of PBS and delivered intravenously (iv.). One mg of rabbit anti-FITC antibody and 250 µg of F(ab¹)₂ ATG per mouse are injected at the times indicated in the brief description of the drawings.

At each time point the mice are anesthetized with ether and bled via the retro-orbital plexus. A 1:10 dilution of blood in glacial acetic acid is used to lyse the red blood cells and leave the PBLs intact. The number of PBLs are then counted in a hemocytometer. Numbers of CD3⁺ cells are determined by multiplying the numbers of PBLs by the percentage of CD3⁺ cells as determined by FACS analysis.

### Results and Comments

*Complexine binding and recognition by anti-FITC antibodies*: In order to determine whether or not the F(ab¹)₂ moiety of the complexine could still bind to cell surface markers after being conjugated to FITC, P815 cells are incubated with the construct. Figure 1 clearly shows that the presence of F(ab¹)₂ ATG with an F/P 1 alone is directly detectable by excitation of the fluorescein portion of the conjugate during FACS analysis. In the absence of complexine on the cells, there is residual binding of the polyclonal rabbit anti-FITC antibodies (rab-a-FITC peak). The higher FITC fluorescence activity seen on the P815 cells indicates that the complexine binds to the cells on its own. The presence of anti-FITC antibodies added to the complexine stained cells could be revealed using an indirect labeling with biotinylated goat anti-rabbit immunoglobulins and streptavidin-FITC. The greatly enhanced signal seen in this way demonstrates the association of the rabbit anti-FITC antibodies with the target P815 cell via the F(ab¹)₂ATG-FITC bridge.

*The complex can initiate complement mediated lysis*: Figures 2A and 2B illustrate the assay developed to evaluate the effect of complement on cells bound to this complexine construct and the anti-FITC antibodies. P815 cells are divided into two groups. One group is stained with F(ab¹)₂ATG-FITC and then mixed with the unstained group. P815 cells stained or unstained with F(ab¹)₂ATG are lysed *in vitro* only after the addition of both the anti-FITC antibodies and complement. The presence of either the complexine or the anti-FITC antibodies alone is not sufficient to induce detectable lysis in this system.

It is interesting to note that lysis does not occur with all F(ab¹)₂ATG-FITC conjugates even in the presence of anti-FITC antibodies. Other F/P ratios of complexine are prepared and assessed for their ability to bind rabbit anti-FITC antibodies and induce lysis in the presence of complement. While a conjugate with an F/P of 6.7 could bind and induce lysis as well as the F/P 1 preparation, a third conjugate with an F/P of 25 is capable only of binding the anti-FITC antibodies, but did not induce any cell lysis after the addition of complement.

*F(ab*^{*1*}*)*_{*2*}*ATG-FITC and anti-FITC antibodies are detectable in vivo after injection*: FACS analysis of PBLs after the i.v. administration of F(ab¹)₂ATG-FITC permits the visualization of the complexine bound to cells *in vivo*. Figure 3 is typical of a FACS profile of PBLs from a complexine treated mouse stained *ex vivo* with anti-CD3. The complexine is fixed on the PBLs *in vivo*. This shows that while the ATG fragment binds to nearly all cells, it preferentially binds to the T-cell population. In this manner the presence of complexine on the cell surface *in vivo* could be followed for more than 48 hours.

In order to obtain high titers of anti-FITC antibodies in mice without having to wait for priming and boosting of the animals with carrier hapten conjugates, we choose to passively transfer polyclonal rabbit anti-FITC antibodies. This also allowed us to track the presence of the injected antibodies by ELISA. Figure 4 presents the data from five mice 48 hours after the injection of the anti-FITC antibodies. The rabbit immunoglobulins are still circulating at this time point, and can be detected for at least 120 hrs after injection.

*Anti-FITC and complexine in vivo eliminates target cells*: Mice which had previously been transfused with anti-FITC antibodies are subsequently injected with the F(ab¹)₂ATG-FITC conjugate 24 hrs later. After an additional 24 hr period, the level of circulating PBLs is evaluated (see Figure 5A). This data is correlated with the FACS evaluation of CD3⁺ percentages among the PBLs and the effect on the absolute numbers of T-cells calculated (see Figure 5B). In both cases there is a significant (p<= 0.02 Scheffe ANOVA) decrease in the numbers of target cells only in the mice receiving both the anti-FITC antibodies and the complexine. This decrease is only seen in the test group after the administration of both components.

The dose of complexine required to achieve the reduction in circulating T-cells can be reduced. *In vivo* titration experiments prove that the quantity of F(ab¹)₂ATG-FITC used can be decreased at least five fold and still deplete the CD3⁺ population of cells as well as the 250 µg dose (see Figure 6). Furthermore, the reduced dose of complexine proved as effective as an equivalent dose of ATG at reducing cell numbers (Figure 7). This demonstrates that the complexine technology appears as effective as conventional means for targeting a cell population *in vivo*.

To explore the possibility that circulating immune complexes (CIC) might be preventing a maximum amount of complexine from reaching the cells *in vivo* in the presence of anti-FITC antibodies, a similar experiment is designed. However, in this instance the F(ab¹)₂-ATG-FITC conjugate is administered prior to the transfusion of anti-FITC antibodies. This ensured that the complexine construct could bind freely to cells *in vivo* without being cleared by the anti-FITC antibodies. With this type of reverse experiment a decrease in the numbers of CD3⁺ cells similar to that seen when the injections are given in the proper sequence is seen. This indicated that there is only a small or no effect due to clearance of the F/P 1 conjugate in the presence of circulating anti-FITC antibodies. *In vitro* formation and assay of the immune complexes formed between the anti-FITC immunoglobulins and the three different F/P ratios in the complexine constructs indicated that the formation of such complexes is readily demonstrable with the F/P 25 and F/P 6.7 preparations, but not the F/P 1 conjugate.

These results show the design and construction of a small conjugate capable of binding to a target cell and bearing a marker recognizable by an immune effector component. The antibodies thus bound via a complexine bridge to the target cell can initiate complement mediated lysis *in vitro*. When the complexine construct has been appropriately made to avoid the formation of insoluble immune complexes, they can mediate the elimination of a target cell population *in vivo*.

In accordance with the subject invention, agents are provided which can specifically bind to a target, e.g. cell, human, bacteria, virus infected or parasitic, or soluble molecule via a specific binding ligand. Agents can be selected which show a low affinity for cells which do not pair with the receptor complementary binding member. When using specific agents which interact with an endogenous effector molecule, one can achieve cytotoxicity toward the target cell following binding of the conjugate to the target cell. By employing agents for the ligand moiety which do not induce a significant immune response, such as molecules which are substantially endogenous or have low immunogenicity, one can avoid an immune response and thus avoid having agents of the immune response destroy or inactivate the therapeutic conjugate. By taking advantage of the preexisting immune response against the selective moiety and because the ligand moiety remains functional, one can use the subject agents on a chronic basis.

### Example 5

### Prolongation of Heterotopic Cardiac Grafts

### Materials and Methods

*Anti-α-Gal antibodies: purification and murine cell recognition*: Human anti-α-Gal antibodies were purified from pooled plasma using a melibiose agarose column (Sigma Chemical Co., St. Louis, MO). Briefly, plasma from normal blood donors was passed over the column, the support washed with PBS, and the bound antibodies eluted by adding 0.1M Tris pH 4.0. Protein concentration in the eluted fractions was measured (BCA test; Pierce, Rockford, IL), and the fractions containing protein were pooled.

Mouse lymph node cells were incubated with 10 µg/ml of the anti α-Gal antibodies or a mix of 10 µg/ml each of human monoclonal antibodies (anti-HLA specific IHB-HU-015 IgG and IHB-HU-007 IgM: SVM-Foundation for the Advancement of Public Health and Environmental Protection, Bilthoven, Netherlands) to control for Fc receptor binding for 20 min on ice. The cells were then washed and incubated with biotinylated goat anti-human IgG and IgM antibodies (Jackson ImmunoResearch, West Grove, PA). After washing, the cells were incubated with streptavidin tandem (Southern Biotechnologies, Birmingham, AL), anti-CD8 phycoerythrin and anti-CD4 FITC (Pharmingen, San Diego, CA), then analyzed by flow cytometry using live gates on a FACScan and the Lysys II software (Becton Dickinson, San Jose, CA).

*FITC conjugate preparation*: KLH, BSA (Sigma Chemical Co., St. Louis, MO) and human IL2 (PeproTech, Inc., Rocky Hill, NJ) were coupled to FITC as follows: 1.25 mg of FITC (fluorescein 5-isothiocyanate isomer 1 Sigma Chemical Co.: stock solution at 20 mg/ml in DMSO) was added to 250 mg of KLH or BSA (20µg FITC/mg protein) in parallel, 125 µg of FITC was added to 250 mg of IL2 (2 µg FITC/mg protein). Each preparation was incubated at room temperature for 30 min in the dark. Excess FITC was removed by passing the solutions over a sephadex G10 column (Pharmacia, Uppsala, Sweden). The protein content was measured using a standard bichinoic acid based test (BCA test: Pierce), and the ratio of FITC: protein (F/P) in each conjugate was determined as described elsewhere (Hudson and Hay, Practical Immunology, Cambridge, MA: Blackwell Scientific Publications, 1989, Fd. 3rd pp. 35). IL2-FITC conjugates having a mean F:P ratio of 1 only were used to avoid the formation of immune complexes *in vivo*. KLH/BSA FITC conjugates of F:P ratios of 10 were used for efficient antibody induction.

*Mice, immunizations, injections, and cardiac allograft*: Groups of 4 BALB/c or C57BL/6 male mice 9-10 weeks of age (Simonsen Laboratories, Gilroy, CA) were used throughout the experiments. BALB/c mice were primed and boosted with injections of 20 µg KLH-FITC prepared in CFA or IFA (Complete and Incomplete Freund's Adjuvants respectively; Sigma Chemical Co.) delivered sub-cutaneously in the right hind footpad. At each time point indicated, the mice were anesthetized with 65% CO₂ and bled via the retroorbital plexus. Serum was separated from the sample and either tested in EUSA or passed over a FITC bound column prepared according to the manufacturers instructions (Pharmalink kit, Pierce). Anti-FITC antibodies were eluted from the column with pH 4.0 Tris-Hcl and extensively dialyzed against PBS pH 7.4.

C57BL/6 mice were heavily anesthetized with Metofane (methoxyflurane, Pittmann-Moore, Mundelein, IL), their hearts removed and the organ flushed with heparinised Ringers lactate. Heterotopic cardiac transplant to BALB/c recipients was performed according to the method of Ono and Lindsey (1969) J. Thorac. Cardiovasc. Surg. **7**:225-229.- Treatment with 50 µg/Kg/day (1µg/mouse) IL2-FITC conjugate or IL2 alone was delivered via the tail veins in 200 µl PBS. Graft survival was evaluated daily by direct palpation. Suspected rejection was confirmed bytopening the peritoneal cavity of the recipient and direct observation of the graft. All animals were treated and maintained in accordance with public health service guidelines.

*Anti-FITC ELISA*: 100 µg of BSA-FITC was coated on 96 well Maxisorp (Nunc, Naperville, IL) plastic plates overnight at 4°C. Unbound sites on the plastic were then saturated with a PBS-5% dehydrated non-fat milk (PBS-milk) solution for 1 hour at 37°C. After washing three times with Plate Wash (SangStat Med. Corp., Menlo Park, CA), serial dilutions of samples in PBS-2.5% milk were added to each well and incubated for 1 hour at 37°C. The plates were again washed, then incubated with an anti-mouse Ig-HRP conjugate (Jackson Immuno Research, West Grove, PA). Following another incubation for 1 hour at 37°C and wash, the presence of mouse anti-FITC antibodies was revealed by adding a 3 mg/ml solution of 9-phenylenediamine dihydrochloride (Sigma Chemical Comp.) in substrate buffer (SangStat Med. Corp.). The enzymatic reaction was stopped after 15 min. by the addition of 100 µl 1 N HCl and the results evaluated at 495nm using an Emax spectrophotometer and SoftMax software (Molecular Devices, Menlo Park, CA). Endpoint dilution titers were evaluated as the reciprocal of the last serum dilution to give optical densities greater than 0.2 (*i*.*e*. above background from non-immunized mice).

*CD25*^{*+*} *cells and IL2-FITC binding*: Mouse CTLL-2 cells (ATCC, Rockville, MD), which require IL2 for growth, were maintained in EL4 conditioned medium. Cells were washed three times in PBS pH 5.0 to remove any unlabeled IL2 from the receptor. Cells were then resuspended in 50 µl of the 2 µg/ml IL2-FITC for 20 min on ice. The presence of bound-labeled cytokine was amplified by indirect staining with affinity purified murine anti-FITC antibodies from KLH-FITC immunized mice followed by biotinylated goat anti-mouse Ig (Pharmingen, San Diego, CA) and streptavidin tandem (Southern Biotechnologies, Birmingham, Al). All samples were analyzed using live gates on a FACScan and the Lysys II software (Becton Dickinson).

*Histology*: The heterotopic heart, spleen, kidney, and thymus from the transplanted test and control mice were collected at the times indicated below. Organs were fixed in buffered 10% formalin until analysis. Sectioning, H&E staining, and double blind evaluation of the sections was performed by CVD Inc. (West Sacramento, CA).

*Mixed Lymphocyte Reaction*: One way C57BU6 stimulator to BALB/c responder mixed lymphocyte reactions (MLR) were prepared according to standard protocols. Briefly, freshly isolated C57BL/6 lymph node cells were inactivated by incubation with 25 µg/ml mitomycin C (Calbiochem, La Jolla, CA). After extensive washing the stimulator cells were mixed 1:1 with responder BALB/c lymph node cells and pipetted into 96 well flat bottomed microtiter plates. Serial dilutions of IL2-FITC or IL2 (PeproTech) were added at the beginning of culture to evaluate their toxicity. After 5 days, 1 µCi of 3H-thymidine was added to each well. Eight hours later the cells were harvested and isotope incorporation evaluated using a TopCount microscintillation counter (Packard, Downers Grove, IL).

*IL2-FITC redirects the natural antibody and prolongs graft survival*: The data demonstrates the therapeutic benefit to redirecting a natural antibody response to target CD25⁺ activated T-cells. Two groups of 4 BALB/c mice were immunized twice with KLH-FITC and had titers of circulating anti-FITC antibody. The immunized mice then received heterotopic cardiac allografts from C57BU6 recipients on day 0. The test group received daily injections of IL2-FITC (F:P=1, 50µg/Kg), for 30 days, and is directly compared to the control group receiving the same dose of IL2 alone. The immunized/IL2-FITC treated test group maintained their grafts for 38.7±7.1 days as opposed to the immunized/IL2 treated group which rejected their grafts on day 10±1.4. Clearly the presence of the hapten recognized by the circulating antibodies fixed to the IL2 ligand prolongs graft survival dramatically. Further specificity controls showed that non immunized/untreated controls rejected their grafts by day 9±0.7, immunized/untreated mice rejected grafts on day 13.6±3.6, and nonimmunized/IL2-FITC treated mice rejected on day 9.4±1.1. All control groups rejected their grafts significantly faster than the immunized/IL2-FITC test population (p≤0.02 in the Mann Whitney test).

*IL2-FITC is not toxic*: The inability of the IL2-FITC construct to prolong graft survival in nonimmunized mice indicates that it is not toxic to proliferating T-cells. To expand on this observation, the effect of the conjugate on proliferating cells in an MLR was evaluated. The corresponding effect of IL2 alone was analyzed for comparison. The IL2-FITC conjugate behaves much like the unlabeled cytokine. At high concentrations (10 µg/ml) both preparations accelerate the MLR and the amount of ³H-thymidine incorporation on day 5 of culture is less because the culture has already peaked. At lower doses, cells cultured in the presence of IL2-FITC or IL2 alone both proliferate equally as well or better (due to the stimulatory effect of the cytokine) than the cultures with no additive. This confirms the *in vivo* observation that the IL2-FITC is not toxic.

Two groups of five BALB/c mice were previously immunized with KLH-FITC and received heterotopic cardiac allograft from C57BL/6 donors on Day 0. On the following day and every day thereafter for 30 days, or until rejection, mice received 50 mg/Kg of IL2-FITC or IL2 alone. Graft survival was assessed daily by palpation through the peritoneum.

Non-immunized/untreated controls rejected their grafts by day 9±0.7 while mice treated in accordance with the subject invention maintained their grafts for 38.7 + 7.1 days, a better than 4 fold increase. The difference between the two groups was significant (p≤0.02). This was achieved solely by use of the subject invention in the absence of other immunosuppressive agents.

It is evident from the above results that the subject invention has application *in vivo*. The presence of circulating antibodies specific for a selective agent is exploited to ablate a specific T cell subpopulation. Circulating antibodies can be raised to the selective agent by immunization, or a selective agent to which there exists high levels of endogenous antibodies can be chosen. The selective agent is targeted to activated T cells by the use of a conjugate to a ligand such as IL-2. Antibodies specific for the selective agent then bind to the activated T cells, thereby activating pathways for ADCC and complement killing. By ablating a subpopulation of activated T cells responding to an allogeneic graft, the lifetime of the graft can be greatly extended. The subject method provides a means of selective immunosuppression, without comprising the pool of non-activated T cells, or other cells of the hematopoietic system, thereby decreasing the undesirable side-effects of the immunosuppression.

The invention now being fully described, it will be apparent to one of ordinary skill in the art that many changes and modifications can be made thereto without departing from the scope of the appended claims.

## Claims

1. A pharmaceutical composition comprising a conjugate for inactivating a target cell in a mammalian host, said conjugate comprising a targeting moiety specific for a surface membrane receptor of said target cell covalently joined to a selective moiety, wherein said selective moiety comprises an α-galactosyl epitope and is capable of binding to an endogenous cytotoxic effector system in the host to form a cell inactivating complex, said effector system comprising antibodies specific for said selective moiety and an antibody dependent cytotoxic system comprising at least one effector agent, whereby when said conjugate is bound to said target cell and said effector agent, said target cell is inactivated.

2. The pharmaceutical composition of claim 1, wherein said targeting moiety is folate.

3. The pharmaceutical composition of claim 1, wherein said targeting moiety is a ligand for a cytokine surface membrane receptor.

4. The pharmaceutical composition of claim 3, wherein said ligand for a cytokine surface membrane receptor is IL-2.

5. A pharmaceutical composition comprising a conjugate for inactivating a target cell in a mammalian host, said conjugate comprising a targeting moiety specific for a surface membrane receptor of said target cell covalently joined to a selective moiety, wherein said targeting moiety is folate and said selective moiety comprises an antigen to which the host has been previously sensitized or to which the host has natural immunity, wherein said selective moiety is capable of binding to an endogenous cytotoxic effector system in the host to form a cell inactivating complex, said effector system comprising antibodies specific for said selective moiety and an antibody dependent cytotoxic system comprising at least one effector agent, whereby when said conjugate is bound to said target cell and said effector agent, said target cell is inactivated.

6. The pharmaceutical composition of claim 5, wherein said selective moiety comprises an epitope selected from the group consisting of a blood group antigen to which antibodies are present in said mammalian host, a xenoantigen to which antibodies are present in said mammalian host, and at least a portion of a protein vaccine.

7. The pharmaceutical composition of claim 5, wherein said selective moiety binds to anti-α-galactosyl antibodies.

8. The pharmaceutical composition of claim 7, wherein said selective moiety is the α-galactosyl epitope.

9. The pharmaceutical composition of claim 5, wherein said selective moiety comprises an antigen selected from the group consisting of an oligosaccharide A antigen, an oligosaccharide B antigen, a vaccine antigen, an anti-idiotype antibody, and a variable region of an anti-idiotype antibody.

10. A pharmaceutical composition comprising a conjugate for inactivating a target cell in a mammalian host, said conjugate comprising a targeting moiety specific for a surface membrane receptor of said target cell joined to a selective moiety, wherein said targeting moiety comprises folate and said selective moiety comprises an epitope selected from the group consisting of α-galactosyl, a blood group antigen to which antibodies are present in said mammalian host, a xenoantigen to which antibodies are present in said mammalian host, and at least a portion of protein vaccine, wherein said selective moiety is capable of binding to an endogenous cytotoxic effector system in the host to form a cell inactivating complex, said effector system comprising antibodies specific for said selective moiety and an antibody dependent cytotoxic system comprising at least one effector agent, whereby when said conjugate is bound to said target cell and said effector agent, said target cell is activated.

## Patentansprüche

1. Pharmazeutische Zusammensetzung, welche ein Konjugat zum Inaktivieren einer Zielzelle in einem Säugetierwirt umfasst, wobei das Konjugat einen ansteuernden Teil umfasst, der spezifisch ist für einen Oberflächenmembranrezeptor der Zielzelle, welcher kovalent verbunden ist mit einem selektiven Teil, worin der selektive Teil ein α-Galaktosyl-Epitop umfasst und an ein endogenes zytotoxisches Effektorsystem im Wirt binden kann unter Bildung eines Zell-inaktivierenden Komplexes, wobei das Effektorsystem Antikörper, welche spezifisch sind für den selektiven Teil, und ein Antikörper-abhängiges zytotoxisches System umfasst, welches mindestens ein Effetoragens umfasst, wobei die Zielzelle inaktiviert wird, wenn das Konjugat an die Zielzelle und das Effektoragens gebunden ist.

2. Pharmazeutische Zusammensetzung nach Anspruch 1, worin der ansteuernde Teil Folat ist.

3. Pharmazeutische Zusammensetzung nach Anspruch 1, worin der ansteuernde Teil ein Ligand für einen Zytokin-Oberflächenmembranrezeptor ist.

4. Pharmazeutische Zusammensetzung nach Anspruch 3, worin der Ligand für einen ZytoKin-Oberflächenmembranrezeptor IL-2 ist.

5. Pharmazeutische Zusammensetzung, welche ein Konjugat zum Inaktivieren einer Zielzelle in einem Säugetierwirt umfasst, wobei das Konjugat einen ansteuernden Teil umfasst, der spezifisch ist für einen Oberflächenmembranrezeptor der Zielzelle, welcher kovalent verbunden ist mit einem selektiven Teil, worin der ansteuernde Teil Folat ist und der selektive Teil ein Antigen umfasst, für welches der Wirt zuvor sensibilisiert worden ist oder gegen welches der Wirt eine natürliche Immunität besitzt, worin der selektive Teil an ein endogenes zytotoxisches Effektorsystem im Wirt binden kann, um einen Zelt-inaktivierenden Komplex zu bilden, wobei das Effektorsystem Antikörper, welche spezifisch sind für den selektiven Teil und ein Antikörper-abhängiges zytotoxisches System umfasst, welches mindestens ein Effetoragens umfasst, wobei die Zielzelle inaktiviert wird, wenn das Konjugat an die Zielzelle und das Effektoragens gebunden ist.

6. Pharmazeutische Zusammensetzung nach Anspruch 5, worin der selektive Teil ein Epitop umfasst, ausgewählt aus der Gruppe bestehend aus einem Blutgruppenantigen, gegen welches Antikörper in dem Säugetierwirt vorhanden sind, einem Xenoantigen, gegen welches Antikörper in dem Säugetierwirt vorhanden sind oder mindestens einem Teil eines Proteinimpfstoffs.

7. Pharmazeutische Zusammensetzung nach Anspruch 5, worin der selektive Teil an Anti-α-Galaktosyl-Antikörper bindet.

8. Pharmazeutische Zusammensetzung nach Anspruch 7, worin der selektive Teil das α-Galaktosyl-Epitop ist.

9. Pharmazeutische Zusammensetzung nach Anspruch 5, worin der selektive Teil ein Antigen umfasst, welches ausgewählt ist aus der Gruppe bestehend aus einem Oligosaccharid A-Antigen, einem Oligosaccharid B-Antigen einem Impfstoffantigen, einem Anti-Idiotyp-Antikörper und einer variablen Region eines Anti-Idiotyp-Antikörpers.

10. Pharmazeutische Zusammensetzung, welche ein Konjugat zum Inaktivieren einer Zielzelle in einem Säugetierwirt umfasst, wobei das Konjugat einen ansteuernden Teil umfasst, der spezifisch ist für einen Oberflächenmembranrezeptor der Zielzelle, welcher mit einem selektiven Teil verbunden ist, worin der ansteuernde Teil Folat umfasst und der selektive Teil ein Epitop umfasst, welches ausgewählt ist aus der Gruppe bestehend aus α-Galaktosyl, einem Blutgruppen-Antigen, gegen welches Antikörper im Säugetierwirt vorliegen, einem Xenoantigen, gegen welches Antikörper in dem Säugetierwirt vorliegen und mindestens einem Teil eines Proteinimpfstoffs, wobei der selektive Teil an ein endogenes zytotoxisches Effektorsystem im Wirt binden kann, um einen Zell-inaktivierenden Komplex zu bilden, wobei das Effektorsystem Antikörper, welche spezifisch sind für den selektiven Teil und ein Antikörper-abhängiges zytotoxisches System umfasst, welches mindestens ein Effektoragens umfasst, wobei die Zielzelle inaktiviert wird, wenn das Konjugat an die Zielzelle und das Effektoragens gebunden ist.

## Revendications

1. Composition pharmaceutique comprenant un conjugué pour désactiver une cellule cible chez un mammifère hôte, ledit conjugué comprenant un groupement ciblant spécifique d'un récepteur de surface membranaire de ladite cellule cible lié de manière covalente à un groupement sélectif, ledit groupement sélectif comprenant un épitope α-galactosyle et étant capable de se lier à un système effecteur cytotoxique endogène dans l'hôte pour former un complexe désactivateur de cellule, ledit système effecteur comprenant des anticorps spécifiques du dit groupement sélectif et un système cytotoxique dépendant de l'anticorps comprenant au moins un agent effecteur, sachant que lorsque ledit conjugué est lié à ladite cellule cible et au dit agent effecteur, ladite cellule cible est désactivée.

2. Composition pharmaceutique selon la revendication 1, dans laquelle ledit groupement ciblant est le folate.

3. Composition pharmaceutique selon la revendication 1, dans laquelle ledit groupement ciblant est un ligand pour un récepteur de cytokine de surface membranaire.

4. Composition pharmaceutique selon la revendication 3, dans laquelle ledit ligand pour un récepteur de cytokine de surface membranaire est IL-2.

5. Composition pharmaceutique comprenant un conjugué pour désactiver une cellule cible chez un mammifère hôte, ledit conjugué comprenant un groupement ciblant spécifique d'un récepteur de surface membranaire de ladite cellule cible lié de manière covalente à un groupement sélectif, ledit groupement ciblant étant le folate et ledit groupement sélectif comprenant un antigène auquel l'hôte a été préalablement sensibilisé ou contre lequel l'hôte dispose d'une immunité naturelle, ledit groupement sélectif étant capable de se lier à un système effecteur cytotoxique endogène dans l'hôte pour former un complexe désactivateur de cellule, ledit système effecteur comprenant des anticorps spécifiques du dit groupement sélectif et un système cytotoxique dépendant de l'anticorps comprenant au moins un agent effecteur, sachant que lorsque ledit conjugué est lié à ladite cellule cible et au dit agent effecteur, ladite cellule cible est désactivée.

6. Composition pharmaceutique selon la revendication 5, dans laquelle ledit groupement sélectif comprend un épitope choisi dans le groupe comprenant un antigène de groupe sanguin contre lequel des anticorps sont présents dans ledit hôte mammifère, un xénoantigène contre lequel des anticorps sont présents dans ledit hôte mammifère et au moins une partie d'un vaccin protéique.

7. Composition pharmaceutique selon la revendication 5, dans laquelle ledit groupement sélectif se lie à des anticorps anti-α-galactosyle.

8. Composition pharmaceutique selon la revendication 7, dans laquelle ledit groupement sélectif est l'épitope α-galactosyle.

9. Composition pharmaceutique selon la revendication 5, dans laquelle ledit groupement sélectif comprend un antigène choisi dans le groupe comprenant un antigène oligosaccharide A, un antigène oligosaccharide B, un antigène de vaccin, un anticorps anti-idiotype et une région variable d'anticorps anti-idiotype.

10. Composition pharmaceutique comprenant un conjugué pour désactiver une cellule cible dans un hôte mammifère, ledit conjugué comprenant un groupement ciblant spécifique d'un récepteur de surface membranaire de ladite cellule cible lié à un groupement sélectif, ledit groupement ciblant comprenant le folate et ledit groupement sélectif comprenant un épitope choisi dans le groupe comprenant le α-galactosyle, un antigène de groupe sanguin contre lequel des anticorps sont présents dans ledit hôte mammifère, un xénoantigène contre lequel des anticorps sont présents dans ledit hôte mammifère, et au moins une partie d'un vaccin protéique, ledit groupement sélectif étant capable de se lier à un effecteur cytotoxique endogène dans l'hôte pour former un complexe désactivateur de cellule, ledit système effecteur comprenant des anticorps spécifiques du dit groupement sélectif et un système cytotoxique dépendant de l'anticorps comprenant au moins un agent effecteur, sachant que lorsque ledit conjugué est lié à ladite cellule et au dit agent effecteur, ladite cellule cible est activée.
